# EUROPÄISCHE PATENTSCHRIFT

(11) **EP 2 534 281 B1**
(45) Veröffentlichungstag und Bekanntmachung des Hinweises auf die Patenterteilung: **09.01.2019**
(21) Anmeldenummer: 11704430.5
(22) Anmeldetag: 08.02.2011
(51) Int. Cl.: C25B 3/02

(54) **VERFAHREN ZUR HERSTELLUNG VON 4-ISOPROPYLCYCLOHEXYLMETHANOL**
METHOD FOR PRODUCING 4-ISOPROPYLCYCLOHEXYLMETHANOL
PROCÉDÉ DE PRÉPARATION DE 4-ISOPROPYLCYCLOHEXYLMÉTHANOL

(30) Priorität: 12.02.2010 EP 10153392
(43) Veröffentlichungstag der Anmeldung: 19.12.2012
(73) Patentinhaber: BASF SE, 67056 Ludwigshafen am Rhein (DE)
(72) Erfinder: STECKER, Florian, 68167 Mannheim (DE); GRIESBACH, Ulrich, 68305 Mannheim (DE); BOCK, Martin, 30167 Hannover (DE); KÖNIGSMANN, Lucia, 70197 Stuttgart (DE)
(74) Vertreter: BASF IP Association
(86) Internationale Anmeldenummer: PCT/EP2011/051770
(87) Internationale Veröffentlichungsnummer: WO 2011/098432

(56) Entgegenhaltungen:
- EP-A1- 0 293 739
- EP-A1- 0 293 739
- EP-A1- 0 293 739
- WO-A1-2009/059944
- WO-A1-2009/059944
- WO-A2-2010/079035
- DE-A1- 3 644 076
- DE-A1- 3 644 076
- DE-A1- 3 644 076
- DE-A1- 4 308 846
- DE-A1- 4 308 846
- DE-A1-102005 029 200
- ES-A6- 2 009 174
- ES-A6- 2 009 174
- KR-A- 20040 072 433
- PETER LOYSON ET AL: "Mechanistic and Kinetic Aspects of the Direct Electrochemical Oxidation of 4-t-Butyltoluene", SOUTH AFRICAN JOURNAL OF CHEMISTRY, Bd. 55, 2002, Seiten 125-131, XP002636871, ISSN: 0379-4350

## Beschreibung

Die vorliegende Erfindung betrifft ein Verfahren zur Herstellung von Isopropylcyclohexylmethanol, wobei man eine Verbindung vom para-Cymol-Typ elektrochemisch methoxyliert, das gebildete Acetal zum Aldehyd hydrolysiert, und anschließend den Aldehyd zur gewünschten Verbindung hydriert.

Es ist bekannt, substituierte Benzaldehyddimethylacetale direkt durch elektrochemische Methoxylierung der entsprechenden Toluole herzustellen. P. Loyson, S. Gouws, B. Barton, M. Ackermann, S. Afr. J. Chem 2004, 57, 53-56 beschreiben ein derartiges Verfahren. Nachteilig an der elektrochemischen Seitenkettenmethoxylierung von Toluolen ist, dass nur mit elektronenschiebenden Resten wie tert-Butyl, Methyl oder Alkoxy substituierte Toluole in wirtschaftlich interessanten Ausbeuten methoxyliert werden können. Reste wie Ethyl, Isopropyl oder Isobutyl wirken zwar ebenfalls elektronenschiebend, ihre benzylischen Protonen können aber ebenfalls bei der elektrochemischen Umsetzung in einer Nebenreaktion durch Methoxygruppen substituiert werden. So lässt sich p-Cymol nicht glatt zum Cuminaldehyd-Dimethylacetal methoxylieren, wie in F. Vaudano, P. Tissot, Electrochimica Acta 2001, 46, 875-880 beschrieben, da auch stets die Isopropylgruppe anteilig methoxyliert wird.

Substituierte Benzaldehyddimethylacetale und die diesen zugrunde liegenden Aldehyde sind wichtige Zwischenprodukte z.B. in der Synthese von Riechstoffen wie z.B. Cyclamenaldehyd, Lysmeral®, Silvial oder 4-Isopropylcyclohexylmethanol (IPCHM), das unter dem Handelsnamen Mayol® vertrieben wird.

Die EP 0 129 795 A2 beschreibt ein Verfahren zur Herstellung von substituierten Benzaldehyddialkylacetalen durch Elektrooxidation von entsprechend substituierten Alkyltoluolen bei der man einen Elektrolyten verwendet, der 50 bis 90 Gew.% an einem entsprechenden Alkanol, 8.5 bis 40 Gew.% an dem Alkyltoluol und 0,01 bis 1,5 Gew.% an einer HO₃S-Gruppen enthaltenden Säure enthält.

Die EP 0 554 564 A1 offenbart ein Verfahren zur Herstellung von substituerten Benzaldehydacetalen, wobei die Substituenten des Aromaten mindestens ein benzylisches Wasserstoffatom aufweisen, durch elektrochemische Oxidation eines entsprechenden Benzylethers in Gegenwart eines entsprechenden Alkanols sowie in Gegenwart eines Hilfselektrolyten, indem man im sauren, neutralen oder schwach basischen Bereich elektrolysiert.

Die EP 0 638 665 A1 offenbart ein Verfahren zur Herstellung von substituierten Benzaldehyddialkylacetalen durch elektrochemische Oxidation entsprechend substituierter Toluolverbindungen, indem man eine substituierte Toluolverbindung in Gegenwart eines Alkanols und eines Hilfselektrolyten in einer Elektrolysezelle oxidiert, die so erhaltene Reaktionslösung außerhalb der Elektrolysezelle auf einen Druck entspannt, der 10 mbar bis 10 bar geringer ist als der Druck in der Elektrolysezelle.

Die EP 0 293 739 offenbart ein Verfahren zur Herstellung von 4-Isopropylcyclohexylmethanol (IPCHM) und dessen Alkylethern durch Kernhydrierung in Gegenwart von Edelmetallen der Gruppe VIII des Periodensystems, wie z.B. Nickel, Palladium, Platin, Rhodium oder Ruthenium ausgehend von 4-(1-Alkoxy-1-methyl-ethyl)-benzaldehyden bzw. den entsprechenden Dialkylacetalen, die auf elektrochemischem Wege aus para-Cymol erhältlich sind. Nachteilig an diesem Verfahren ist, dass der verwendete Einsatzstoff 4-(1-Alkoxy-1-methyl-ethyl)-benzaldehyd in einem separaten Verfahrensschritt aus 4-(1-Alkoxy-1-methyl-ethyl)-toluol und dieses wiederum in einem separaten Verfahrensschritt aus para-Cymol hergestellt werden muss.

Die WO 2010/079035 offenbart ein Verfahren zu Herstellung von 4-Isopropylcyclohexylmethanol durch katalytische Hydrierung an einem Festbettkatalysator, enthaltend als Aktivmetall Ruthenium aufgebracht auf einen Träger enthaltend Siliziumdioxid als Trägermaterial.

Die DE 24 27 609 offenbart alicyclische Cyclohexylmethanole und deren Ether oder Ester, die in 4-Position einen Isopropyl- oder Isopropenylrest aufweisen, sowie deren Verwendung als Riech- oder Geschmacksstoffe. Darüber hinaus offenbart die Schrift Verfahren zur Herstellung der genannten Verbindungen durch katalytische Hydrierung von entsprechend ungesättigten Ausgangsverbindungen. Beispielhaft wird die Herstellung von 4-Isopropylcyclohexylmethanol durch katalytische Hydrierung von Cuminaldehyd in 1,2-Dimethoxyethan als Lösungsmittel und in Gegenwart eines Ruthenium-Kohle-Katalysators mit einem Rutheniumgehalt von 5% beschrieben. Die Umsetzung wurde bei einem Druck von 100 Atmosphären und bei einer Temperatur von 130°C durchgeführt und lieferte nach fraktionierter Destillation des Rohproduktes 4-Isopropylcyclohexylmethanol in Form eines Gemisches von 70:30 Gewichtsteilen der cis- und trans-Isomeren.

DE 36 44 076 beschreibt neue Benzaldehydderivate, ihre Herstellung und Verwendung. WO 2009/059944 beschreibt ein elektrochemisches Verfahren zur Herstellung von Benzaldehyddimethylacetalen.

Aufgabe der vorliegenden Erfindung war es, ein einfaches und für die großtechnische Herstellung brauchbares Verfahren zur Herstellung von 4-Isopropylcyclohexylmethanol (IPCHM) bereitzustellen, welches die Nachteile der bekannten Verfahren nicht aufweist.

Die Aufgabe wurde dadurch gelöst, dass man z.B. eine Mischung aus Cuminaldehyd und 4-(1-Alkoxy-1-methyl-ethyl)-benzaldehyd (Alkoxycuminaldehyd), die durch elektrochemische Methoxylierung von para-Cymol und Hydrolyse der entsprechenden Alkylacetale erhalten werden kann, in Gegenwart von Edelmetallen der Gruppe VIII des Periodensystems und in Gegenwart von Wasserstoff umsetzt.

Gegenstand der vorliegenden Erfindung ist somit ein Verfahren zur Herstellung von 4-Isopropylcyclohexylmethanol der Formel (I) 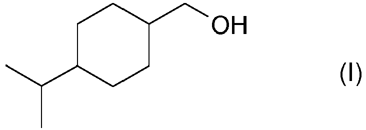 umfassend die Schritte
a) elektrochemische anodische Methoxylierung dadurch gekennzeichnet, dass man
   die elektrochemische anodische Methoxylierung mit mindestens zwei Verbindungen der Formeln (IIa), (IIb), (IIIa) und/oder (IIIb) 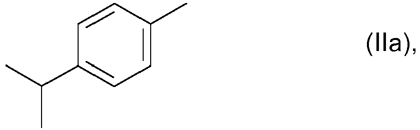 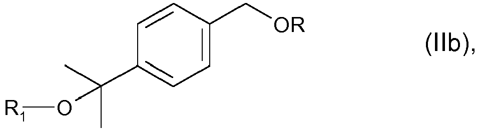 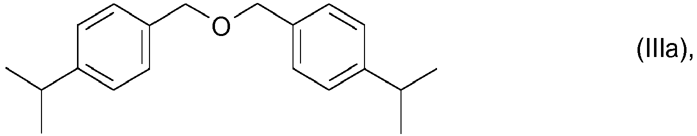 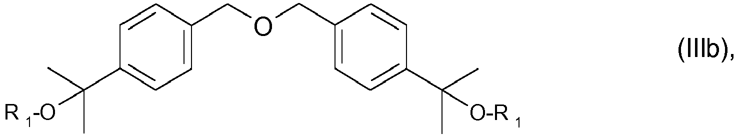 so durchführt, dass eine Mischung der Diacetale der Formeln (IVa) und (IVb) 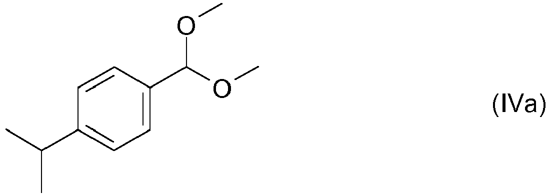 und 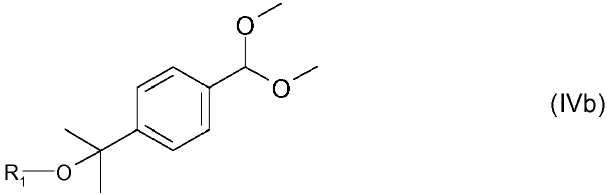 gebildet wird, wobei R₁ in den Formeln (IIb), (IIIb) und (IVb) einen geradkettigen oder verzweigten Alkylrest mit 1 bis 6 Kohlenstoffatomen bedeutet, und R in Formel (IIb) Methyl oder -C(O)R' ist, und R' einen geradkettigen oder verzweigten Alkylrest mit 1 bis 6 Kohlenstoffatomen bedeutet,
b) die Mischung der Diacetale der Formeln (IVa) und/oder (IVb) hydrolysiert unter Bildung der Aldehyde der Formeln (Va) und/oder (Vb) 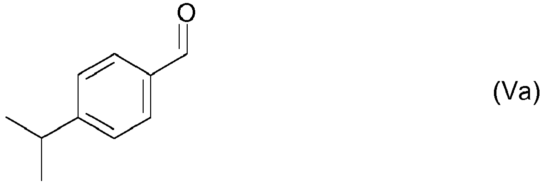 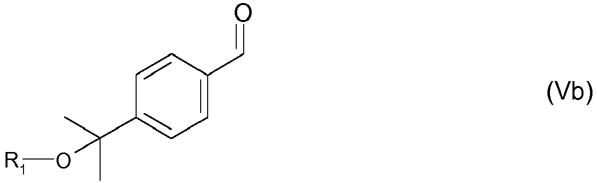 wobei R₁ einen geradkettigen oder verzweigten Alkylrest mit 1 bis 6 Kohlenstoffatomen bedeutet, und
c) das Gemisch der Aldehyde der Formeln (Va) und (Vb) hydriert zu 4-Isopropylcyclohexlmethanol der Formel (I) in Gegenwart von Wasserstoff oder einem wasserstoffhaltigen Gas an einem Katalysator der als Aktivmetall mindestens ein Edelmetall der Gruppe VIII des Periodensystems auf einem Träger enthält.

Das gewünschte Endprodukt des erfindungsgemässen Verfahrens ist in der Regel eine Mischung aus cis- und trans-Isopropylcyclohexylmethanol (IPCHM) der Formeln (la) und (Ib) 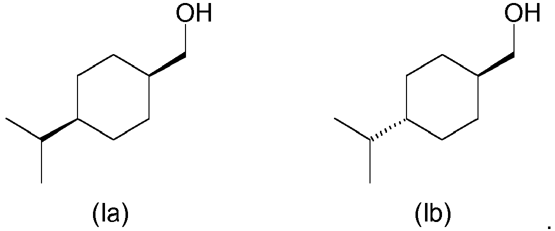 In der Ausgangsverbindung der Formel (II), worin R' und R₁ unabhängig voneinander einen geradkettigen oder verzweigten Alkylrest mit 1 bis 6 Kohlenstoffatomen bedeuten, kommen als Alkylreste z.B. Methyl, Ethyl, Propyl, Isopropyl, n-Butyl, Isobutyl, tert-Butyl, sek-Butyl, Pentyl und Hexyl in Betracht.

Die Hydrierung wird in Gegenwart von Wasserstoff bzw. wasserstoffhaltigen Gasgemischen durchgeführt. Solche Gasgemische können neben Wasserstoff Gase wie Stickstoff oder kohlenwasserstoffhaltige Reformer-Abgase umfassen, nicht jedoch Katalysatorgifte wie Kohlenmonoxid, Schwefelwasserstoff oder andere schwefelhaltige Gase. Bevorzugt setzt man reinen Wasserstoff (Reinheit ≥ 99,9 Vol.-%, besonders ≥ 99,95 Vol.-%, insbesondere ≥ 99,99 Vol.-%) ein.

Die Hydrierung kann wahlweise in einem Lösungsmittel oder unverdünnt durchgeführt werden. Geeignete Lösungsmittel sind beispielsweise Alkohole wie Methanol oder Ethanol, Ether wie Tetrahydrofuran oder Dioxan, Kohlenwasserstoffe wie Pentan und Säuren wie Essigsäure. Bevorzugt wird die Reaktion ohne Lösungsmittel durchgeführt. Die erfindungsgemäße Hydrierung kann kontinuierlich oder diskontinuierlich durchgeführt werden.

Die Hydrierung findet in Gegenwart von Edelmetallen der Gruppe VIII des Periodensystems, wie z.B. Nickel, Palladium, Platin, Rhodium oder Ruthenium, vorzugsweise Rhodium oder Ruthenium, besonders bevorzugt Ruthenium, und in Gegenwart von Wasserstoff unter Druck, insbesondere unter Wasserstoffdrücken von 30-300 bar, vorzugsweise von 100 bis 200 bar, bei vorzugsweise höheren Temperaturen, bevorzugt bei 50°C bis 250°C, besonders bevorzugt bei 80 bis 200°C und ganz besonders bevorzugt bei 120 bis 160°C, statt.

Eine bevorzugte Ausführungsform des erfindungsgemässen Verfahrens ist dadurch gekennzeichnet, dass man bei der Hydrierung als Aktivmetall mindestens ein Edelmetall der Gruppe VIII des Periodensystems, vorzugsweise Rhodium und Ruthenium, ganz besonders bevorzugt Ruthenium, entweder alleine oder zusammen mit mindestens einem weiteren Metall der Nebengruppen IB (z.B. Kupfer, Silber, Gold), VIIB (z.B. Mangan, Rhenium) oder VIII des Periodensystems der Elemente (z.B. Nickel, Palladium, Platin) (CAS-Version), einsetzt. Das Edelmetall wird auf einen Träger aufgebracht, der vorzugsweise Siliziumdioxid und/oder Aluminiumoxid enthält, ganz besonders bevorzugt ist Siliziumdioxid als Trägermaterial. Die Menge des Aktivmetalls ist < 1 Gew.-% bezogen auf das Gesamtgewicht des Katalysators, und mindestens 60 Gew.-% des Aktivmetalls liegt in der Schale des Katalysators bis zu einer Eindringtiefe von 200 µm vor, ermittelt mittels SEM-EPMA (EDXS). Ganz besonders bevorzugt verwendet man ein Edelmetall der Gruppe VIII des Periodensystems allein, insbesondere Ruthenium.

Eine weitere bevorzugte Ausführungsform des erfindungsgemässen Verfahrens ist dadurch gekennzeichnet, dass man bei der Hydrierung als Aktivmetall mindestens ein Edelmetall der Gruppe VIII des Periodensystems, vorzugsweise Rhodium und Ruthenium, entweder alleine oder zusammen mit mindestens einem weiteren Metall der Nebengruppen IB, VIIB oder VIII des Periodensystems der Elemente, aufgebracht auf einen Träger enthaltend Siliziumdioxid als Trägermaterial, einsetzt, wobei die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen.

Eine weitere bevorzugte Ausführungsform der Hydrierungsstufe des erfindungsgemässen Verfahrens ist dadurch gekennzeichnet, dass die Hydrierung in Gegenwart der Aldehyde der Formeln (Va) und (Vb), wobei R₁ Methyl bedeutet, und ferner in Gegenwart der entsprechenden Alkohole, 4-Isopropylbenzylalkohol und 4-(1-Methoxy-1-methyl-ethyl)-benzylalkohol, ausgeführt werden kann.

Eine weitere bevorzugte Ausführungsform des erfindungsgemässen Verfahrens ist dadurch gekennzeichnet, dass man einen Festbettkatalysator einsetzt, der bezogen auf das Gesamtgewicht des fertigen Katalysators einen Aktivgehalt, insbesondere eine Rutheniumgehalt, von 0,1 bis 0,5 Gew.-% aufweist.

Als Ausgangsverbindung zur Durchführung des erfindungsgemäßen Verfahrens dient die Verbindung para-Cymol der Formel (IIa) 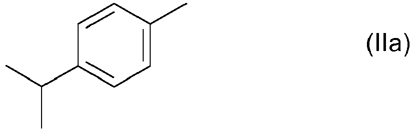 und/oder 4-Isopropyl-benzylverbindungen der Formel (IIb), 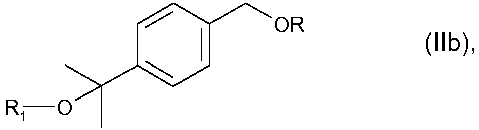 wobei R₁ in Formel (IIb) einen geradkettigen oder verzweigten Alkylrest mit 1 bis 6 Kohlenstoffatomen bedeutet, und R in Formel (IIb) Methyl oder -C(O)R' ist, und R' einen geradkettigen oder verzweigten Alkylrest mit 1 bis 6 Kohlenstoffatomen bedeutet, d.h. z.B. 4-Isopropyl-methylbenzylether und/oder die Ester der Formel (IIb), und/oder Di-(4-isopropylbenzyl)ether der Formel (IIIa) 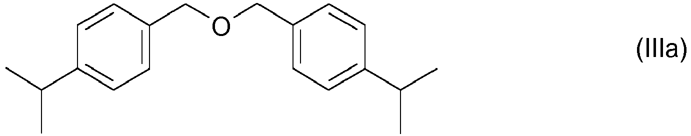 und/oder der Di-(4-isopropylbenzyl)ether der Formel (IIIb) 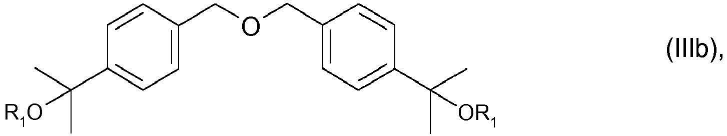 worin R₁ die unter Formel (IIb) angegebene Bedeutung hat.

Die genannten Verbindungen werden im Rahmen des erfindungsgemäßen Verfahrens in Form jedweder Gemische von zwei oder allen drei der genannten Verbindungen eingesetzt werden. Besonders bevorzugt wird die elektrochemische anodische Methoxylierung in Gegenwart der Verbindung der Formel (IIa) durchgeführt.

Das erfindungsgemäße Verfahren ist dadurch gekennzeichnet, dass man
a) die elektrochemische anodische Methoxylierung mit mindestens zwei Verbindungen der Formeln (IIa), (IIb), (IIIa) und/oder (IIIb) 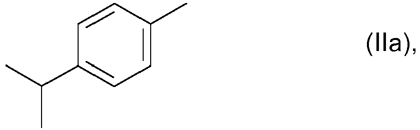 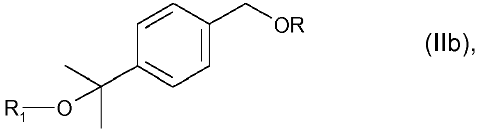 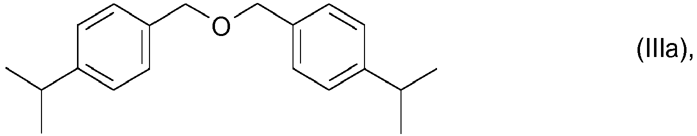 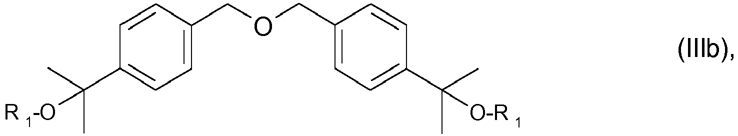 so durchführt, dass eine Mischung der Diacetale der Formeln (IVa) und (IVb) 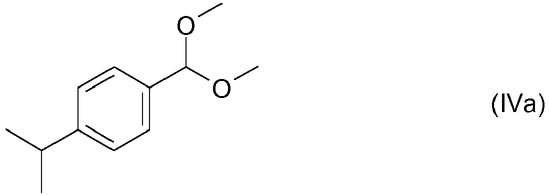 und 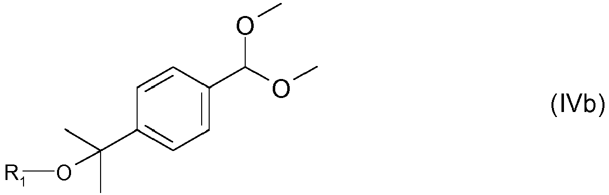 gebildet wird, wobei R₁ in den Formeln (IIb), (IIIb) und (IVb) einen geradkettigen oder verzweigten Alkylrest mit 1 bis 6 Kohlenstoffatomen bedeutet, und R in Formel (IIb) Methyl oder -C(O)R' ist, und R' einen geradkettigen oder verzweigten Alkylrest mit 1 bis 6 Kohlenstoffatomen bedeutet,
b) die Mischung der Diacetale der Formeln (IVa) und/oder (IVb) hydrolysiert unter Bildung der Aldehyde der Formeln (Va) und/oder (Vb) 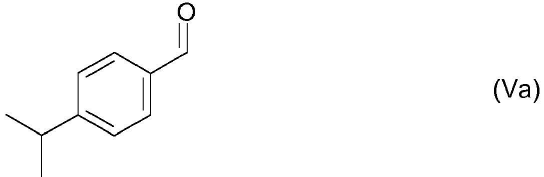 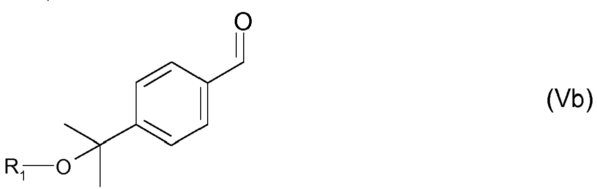 wobei R₁ einen geradkettigen oder verzweigten Alkylrest mit 1 bis 6 Kohlenstoffatomen bedeutet, und
c) das Gemisch der Aldehyde der Formeln (Va) und (Vb) hydriert zu 4-Isopropylcyclohexlmethanol der Formel (I) in Gegenwart von Wasserstoff oder einem wasserstoff-haltigen Gas an einem Katalysator der als Aktivmetall mindestens ein Edelmetall der Gruppe VIII des Periodensystems auf einem Träger enthält.

Im Rahmen einer besonders bevorzugten Ausführungsform des erfindungsgemäßen Verfahrens setzt man para-Cymol der Formel (IIa) als Ausgangsstoff ein. Im Verlauf der erfindungsgemäßen elektrochemischen anodischen Methoxylierung können sich daraus zunächst die genannten Verbindungen der Formeln (II),
Formel (II) 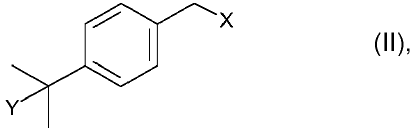 worin X Wasserstoff oder den Rest -O-R und Y Wasserstoff oder den Rest -O-R₁ bedeutet, wobei R Methyl oder -C(O)R' ist, und R' und R₁ unabhängig voneinander einen geradkettigen oder verzweigten Alkylrest mit 1 bis 6 Kohlenstoffatomen bedeuten,
insbesondere 4-Isopropyl-benzyl-methylether der Formel (IIc) 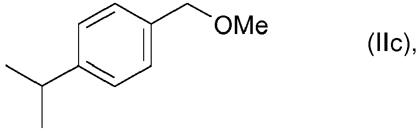 worin Me Methyl bedeutet, und 4-(1-Alkoxy-1-methyl-ethyl)-benzyl-methylether der Formel (IIe) 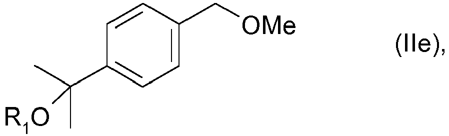 worin Me Methyl bedeutet und R₁ einen geradkettigen oder verzweigten Alkylrest mit 1 bis 6 Kohlenstoffatomen bedeutet, bilden, die dann ihrerseits unter den Reaktionsbedingungen zum gewünschten Gemisch der Alkylacetale der Formel (IVa) und (IVb) 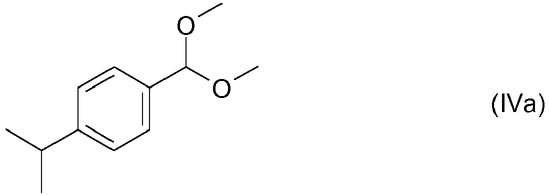 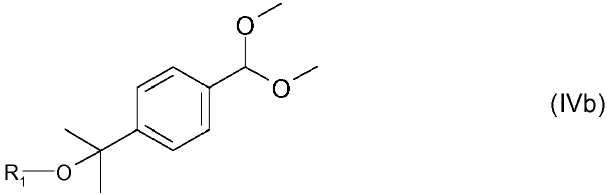 abreagieren bzw. weiter umgesetzt werden. wobei R₁ einen geradkettigen oder verzweigten Alkylrest mit 1 bis 6 Kohlenstoffatomen bedeutet.

4-lsopropylbenzylmethylether der Formel (IIc) ist bekannt und kann wie beispielsweise in WO 2009059941 bzw. WO 2009059944 beschrieben durch Methoxymethylierung von Cumol mit Formaldehyd-Dimethylacetal an einem Zeolith-Katalysator hergestellt werden.

Die Herstellung substituierter Benzylmethylether allgemein aus Alkylbenzolen (Toluol, Ethylbenzol, Isobutylbenzol, Cumol, tert.-Butylbenzol etc.) durch Umsetzung mit Formaldehyd-Dimethylacetal an einem Zeolith-Katalysator ist in DE 199 04 900 beschrieben. Bei niedrigen Umsätzen (< 30%) werden gute Produktselektivitäten erhalten, bei höheren Umsätzen überwiegt die Reaktion zum Diarylmethan. Weitere dem Fachmann bekannte Möglichkeiten zur Herstellung des Benzylmethylethers der Formel (IId) sind die Methylierung von 4-Isopropyl-benzylalkoholen oder die Umsetzung von 4-Isopropyl-benzylhalogeniden mit Methanol bzw. Methanolaten in einer Williamson-Ethersynthese.

Der Dibenzylether der Formel (IIIa) und/oder (IIIb) kann beispielsweise durch Umsetzung des entsprechend substituierten Benzylalkohols der Formel (IX) 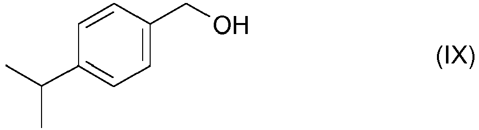 mit dem entsprechend substituierten Benzylhalogenid der allgemeinen Formel (X) 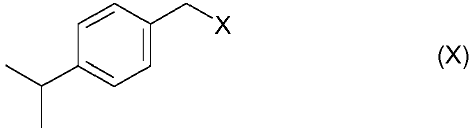 hergestellt werden, worin X ein Halogen aus der Gruppe Chlor, Brom oder lod bedeutet. Dabei wird meist zur Deprotonierung der Alkohole in Gegenwart von Basen gearbeitet (Fileti, Gazz. Chim. Ital. 1884, 14, 498-501). Die Ethersynthese kann auch durch sauer katalysierte Kondensation zweier Moleküle Benzylalkohol ausgeführt werden (Fileti, Gazz. Chim. Ital. 1882, 12, 501; F. Shirini, M.A. Zolfigol, K. Mohammadi, Phosphorus, Sulfur Silicon Relat. Elem. 2003, 178 (11), 2357-2362). Für diese Reaktion gibt es zahlreiche weitere Beispiele in der Literatur.

Der Di-(4-isopropyl-benzyl)ether der Formel (IIIa) und/oder (IIIb) kann auch durch Funktionalisierung von unsubstituiertem Dibenzylether nach einschlägigen Verfahren, die dem Fachmann bekannt sind, wie elektrophile aromatische Substitution oder Friedel-Crafts-Alkylierung, hergestellt werden.

Das Auftreten von Alkylbenzylmethylethern als Zwischenstufen der elektrochemischen Methoxylierung von Alkyltoluolen, so als Zwischenstufen der Methoxylierung von p-tert-Butyltoluol bzw. p-Xylol, ist in P. Loyson, S. Gouws, B. Zeelie, S. Afr. J. Chem., 2002, 55, 125-131 bzw. P. Loyson, S. Gouws, B. Barton, M. Ackermann, S. Afr. J. Chem., 2004, 57, 53-56 beschrieben. Der Eintritt der ersten Methoxygruppe ist dabei der geschwindigkeitsbestimmende Schritt, der infolge dessen mit nur mäßiger Ausbeute verläuft.

Bei der elektrochemischen Methoxylierung des Dibenzylethers der Formel (IIIa) und/oder (IIIb) wird direkt das Benzaldehyd-Dimethylacetale der Formel (IVa) bzw (IVb) erhalten, wobei die Dibenzylether-Bismethoxylierungs-Zwischenstufe (IX) durchlaufen wird. Diese ist jedoch unter den Reaktionsbedingungen nicht beständig und setzt sich mit Methanol unter Freisetzung von Wasser zur Verbindung der Formel (IVa) bzw (IVb) um. 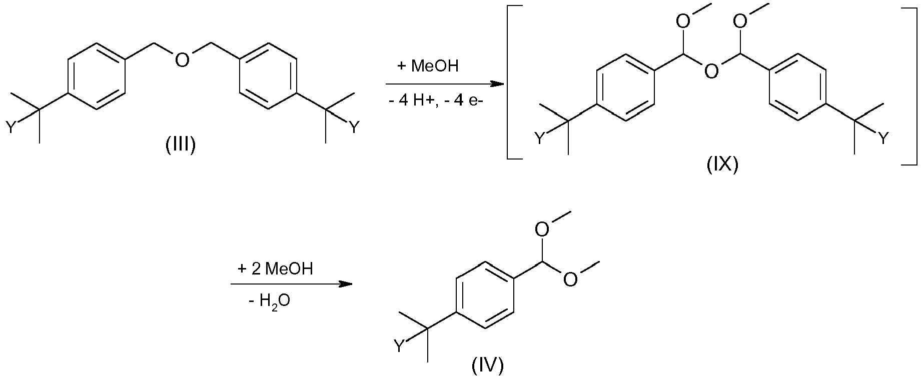 worin Y Wasserstoff oder den Rest -O-R₁ bedeutet, wobei R₁ einen geradkettigen oder verzweigten Alkylrest mit 1 bis 6 Kohlenstoffatomen bedeutet.

Die Elektrolyselösung enthält im Rahmen des erfindungsgemäßen Verfahrens neben den gewählten Ausgangsstoffen der Formeln (IIa) und/oder (IIb) und/oder (IIIa) und/oder (IIIb) mindestens Methanol sowie mindestens ein Leitsalz.

Bei den Leitsalzen, die in der Elektrolyselösung enthalten sein können, handelt es sich im Allgemeinen um Alkali-, Tetra(C₁- bis C₆-alkyl)ammonium-, bevorzugt Tri(C₁- bis C₆-alkyl)methylammoniumsalze. Als Gegenionen kommen Sulfat, Hydrogensulfat, Alkylsulfate, Arylsulfate, Alkylsulfonate, Arylsulfonate, Halogenide, Phosphate, Carbonate, Alkylphosphate, Alkylcarbonate, Nitrat, Alkoholate, Tetrafluorborat oder Perchlorat in Betracht.

Weiterhin kommen die von den vorstehend genannten Anionen abgeleiteten Säuren als Leitsalze in Betracht, also zum Beispiel Schwefelsäure, Sulfonsäuren sowie Carbonsäuren.

Daneben eignen sich als Leitsalze auch ionische Flüssigkeiten. Geeignete ionische Flüssigkeiten sind beschrieben in "Ionic Liquids in Synthesis", Hrsg. Peter Wasserscheid, Tom Welton, Verlag Wiley VCH, 2003, Kap. 1 bis 3 sowie in der DE-A 102004011427.

Bevorzugte Leitsalze sind im Rahmen des erfindungsgemäßen Verfahrens Methyltributylammoniummethylsulfat, Methyltriethylammoniummethylsulfat, Natriummethylsulfonat, Natriumethylsulfonat und Schwefelsäure, insbesondere bevorzugt Natriummethylsulfonat , Methyltributylammoniummethylsulfat und Methyltriethylammoniummethylsulfat und/oder Schwefelsäure, noch mehr bevorzugt Methyltributylammoniummethylsulfat und Methyltriethylammoniummethylsulfat und ganz besonders bevorzugt Methyltributylammoniummethylsulfat. Die genannten Leitsalze, insbesondere Methyltributylammoniummethylsulfat und Methyltriethylammoniummethylsulfat können alleine oder in Form von Gemischen untereinander eingesetzt werden.

Im Rahmen einer bevorzugten Ausführungsform des erfindungsgemäßen Verfahrens setzt man als Leitsalz Methyltributylammoniummethylsulfat und/oder Methyltriethylammoniummethylsulfat ein. Insbesondere bevorzugt setzt man als Leitsalz Methyltributylammoniummethylsulfat ein. Wiederum bevorzugt setzt man die genannten Leitsalze alleine oder in Form eines Gemisches zweier verschiedener Leitsalze, bevorzugt jedoch alleine ein.

Im Rahmen einer vorteilhaften Ausführungsform des erfindungsgemäßen Verfahrens wählt man die Konzentration des Leitsalzes in der Elektrolyselösung im Bereich von 0,1 bis 20 Gewichtsprozent (Gew.-%), bevorzugt im Bereich von 0,2 bis 15 Gew.-%, noch mehr bevorzugt von 0,25 bis 10 Gew.-%, noch mehr bevorzugt von 0,5 bis 7,5 Gew.-% und insbesondere bevorzugt im Bereich von 1 bis 5 Gew.-%. Eine ganz besonders bevorzugte Ausführungsform des erfindungsgemässen Verfahrens ist dadurch gekennzeichnet, dass man als Leitsalz Methyltributylammoniummethylsulfat, Methyltriethylammoniummethylsulfat und/oder Schwefelsäure, vorzugsweise Methyltributylammonium-methylsulfat, einsetzt und die Konzentration des Leitsalzes in der Elektrolyselösung im Bereich von 0,1 bis 20 Gewichtsprozent (Gew.-%) wählt.

Eine weitere bevorzugte Ausführungsform des erfindungsgemäßen Verfahrens ist dadurch gekennzeichnet, dass man die elektrochemische anodische Methoxylierung bei einer Temperatur der Elektrolyselösung im Bereich von 35 bis 70°C, bevorzugt im Bereich von 45 bis 60°C durchführt.

Darüber hinaus führt man das erfindungsgemäße Verfahren bevorzugt so durch, dass man die elektrochemische anodische Methoxylierung bei einem Absolutdruck im Bereich von 500 bis 100000 mbar, bevorzugt bei einem Absolutdruck im Bereich von 1000 bis 4000 mbar durchführt.

Gegebenenfalls setzt man der Elektrolyselösung weitere übliche Lösungsmittel (Cosolventien) zu. Dabei handelt es sich um die in der organischen Chemie allgemein üblichen inerten Lösungsmittel mit einem hohen Oxidationspotential. Beispielhaft genannt seien Dimethylcarbonat oder Propylencarbonat. Im Rahmen einer bevorzugten Ausführungsform führt man das erfindungsgemäße Verfahren daher in Gegenwart von Dimethylcarbonat und/oder Propylencarbonat als Cosolventien durch.

Als Cosolvens ist grundsätzlich auch Wasser geeignet, der Anteil von Wasser im Elektrolyten beträgt bevorzugt weniger als 20 Gew.-%.

Eine besonders bevorzugte Ausführungsform des erfindungsgemässen Verfahrens ist dadurch gekennzeichnet, dass man
a) die elektrochemische anodische Methoxylierung mit mindestens zwei Verbindungen der Formeln (IIa), (IIb) (IIIa) und/oder (IIIb) 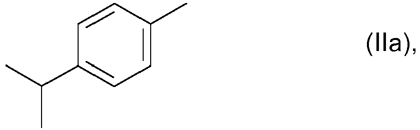 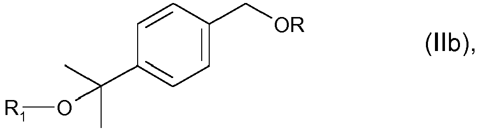 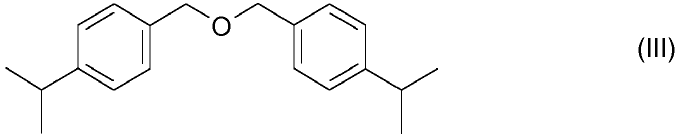 so durchführt, dass eine Mischung der Diacetale der Formeln (IVa) und (IVb) 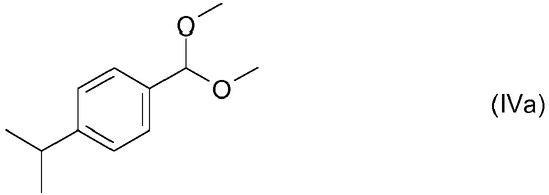 und 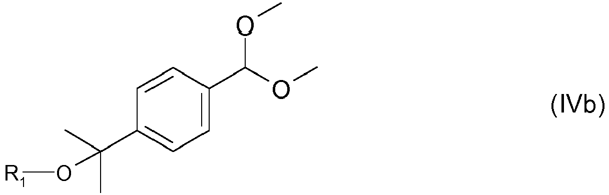 gebildet wird, wobei R₁ in den Formeln (IIb), (IIIb) und in Formel (IVb) einen geradkettigen oder verzweigten Alkylrest mit 1 bis 6 Kohlenstoffatomen bedeutet, und R in Formel (IIb) Methyl oder -C(O)R' ist, und R' einen geradkettigen oder verzweigten Alkylrest mit 1 bis 6 Kohlenstoffatomen bedeutet,
b) die Mischung der Diacetale der Formeln (IVa) und/oder (IVb) hydrolysiert unter Bildung der Aldehyde der Formeln (Va) und/oder (Vb) 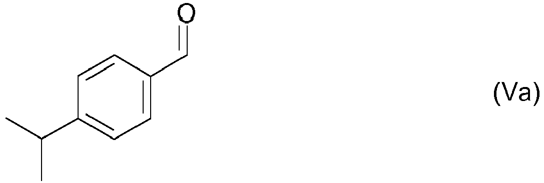 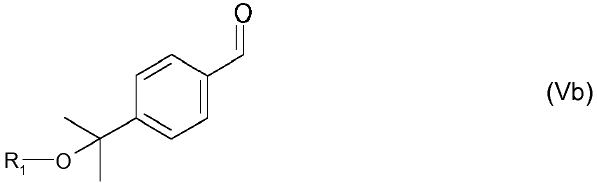 wobei R₁ einen geradkettigen oder verzweigten Alkylrest mit 1 bis 6 Kohlenstoffatomen bedeutet, und
c) das Gemisch der Aldehyde der Formeln (Va) und (Vb) hydriert zu 4-Isopropylcyclohexylmethanol der Formel (I) in Gegenwart von Wasserstoff oder einem wasserstoffhaltigen Gas an einem Katalysator der als Aktivmetall Ruthenium auf einem Träger, der vorzugsweise Siliciumdioxid oder Aluminiumoxid, und ganz besonders bevorzugt Siliciumdioxid enthält.

Die elektrochemische Stufe des erfindungsgemäßen Verfahrens kann in allen üblichen geteilten oder ungeteilten Elektrolysezellentypen durchgeführt werden. Es kann mit gutem Erfolg sowohl diskontinuierlich als auch kontinuierlich durchgeführt werden. Im Rahmen einer bevorzugten Ausführungsform führt man elektrochemische anodische Methoxylierung kontinuierlich durch. Vorzugsweise arbeitet man kontinuierlich mit ungeteilten Durchflusszellen.

Ganz besonders geeignet sind bipolar geschaltete Kapillarspaltzellen oder Plattenstapelzellen, bei denen die Elektroden als Platten ausgestaltet sind und planparallel angeordnet sind (Ullmann's Encyclopedia of Industrial Chemistry, 1999 electronic release, Sixth Edition, VCH-Verlag Weinheim, Volume Electrochemistry, Chapter 3.5 special cell designs sowie Chapter 5, Organic Electrochemistry, Subchapter 5.4.3.2 Cell Design). Als Elektrodenmaterial sind Edelmetalle wie Platin, Mischoxidelektroden wie RuOxTiOx (sogenannte DSA-Elektroden) oder kohlenstoffhaltige Materialien wie Graphit, Glassy Carbon oder Diamantelektroden bevorzugt. Ganz besonders bevorzugt werden Graphitelektroden eingesetzt. Im Rahmen einer bevorzugten Ausführungsform führt man das erfindungsgemäße Verfahren kontinuierlich und unter Verwendung einer Plattenstapelzelle durch.

Die Stromdichten, bei denen man das Verfahren durchführt, betragen im Allgemeinen 1 bis 1000 mA/cm², bevorzugt 10 bis 100 mA/cm². Besonders bevorzugt wird das Verfahren bei Stromdichten zwischen 10 und 50 mA/cm² durchgeführt. Im Allgemeinen wird bei Normaldruck gearbeitet. Höhere Drücke werden bevorzugt dann angewandt, wenn bei höheren Temperaturen gearbeitet werden soll, um ein Sieden der Ausgangsverbindungen bzw. des Lösungsmittels zu vermeiden.

Als Anodenmaterialien eignen sich beispielsweise Edelmetalle wie Platin oder Metalloxide wie Ruthenium oder Chromoxid oder Mischoxide des Typs RuOₓ, TiOₓ sowie Diamantelektroden. Bevorzugt sind Graphit oder Kohleelektroden.

Als Kathodenmaterialien kommen beispielsweise Eisen, Stahl, Edelstahl, Nickel oder Edelmetalle wie Platin sowie Graphit oder Kohlematerialien sowie Diamantelektroden in Betracht. Bevorzugt ist das System Graphit als Anode und Kathode sowie Graphit als Anode und Nickel, Edelstahl oder Stahl als Kathode.

Eine besonders bevorzugte Ausführungsform des erfindungsgemäßen Verfahren ist dadurch gekennzeichnet, dass man die elektrochemische anodische Methoxylierung bei einer Temperatur der Elektrolyselösung im Bereich von 35 bis 70°C, bei einem Absolutdruck im Bereich von 500 bis 100000 mbar und bei einer Stromdichte im Bereich von 10 bis 100 mA/cm² durchführt.

Nach Beendigung der Reaktion wird die Elektrolytlösung nach allgemeinen Trennmethoden aufgearbeitet. Hierzu wird die Elektrolyselösung im Allgemeinen zunächst destilliert und die einzelnen Verbindungen werden in Form von unterschiedlichen Fraktionen getrennt gewonnen. Eine weitere Reinigung kann beispielsweise durch Kristallisation, Extraktion, Destillation oder chromatographisch erfolgen.

Die genannte Hydrolyse kann allgemein nach für den Fachmann an sich bekannten Verfahren, beispielsweise durch einfaches Inkontaktbringen der Verbindung der Formel (IVa) und (IVb) mit Wasser oder einer Säure wie beispielsweise verdünnter Salz-, Schwefel- oder auch Essigsäure bewerkstelligt werden.
Eine besonders bevorzugte Ausführungsform des erfindungsgemässen Verfahrens ist dadurch gekennzeichnet, dass das Massenverhältnis der beiden Aldehyde der Formeln (Va) und (Vb) zwischen 0,2 und 10 liegt.

Die Erfindung wird durch die folgenden Beispiele näher veranschaulicht, ohne sie darauf zu beschränken. Die Abkürzung VE-Wasser bedeutet vollentsalztes Wasser.

### Beispiel 1: Hydrierung einer Mischung aus Cuminaldehyd (Va) und Methoxycuminaldehyd (Vb)

In einem 300 mL Druckreaktor wurden 3,5 g eines gemäß DE102005029200A1 hergestellten Katalysators in einem Katalysator-Einsatzkorb vorgelegt und mit 20 g Methoxycuminaldehyd (Formel Vb) /Cuminaldehyd (Formel Va) 90%ig im Verhältnis 1:1 (in 80 g THF; 20 Gew.-%) versetzt. Die Hydrierung wurde mit reinem Wasserstoff bei einem konstanten Druck von 200 bar und einer Temperatur von 180°C durchgeführt. Es wurde 15 Stunden hydriert. Der Reaktor wurde anschließend entspannt. Der Austrag wurde gaschromatographisch untersucht (GC-Säule: DB-Wax, Länge 30 m, Schichtdicke 0,25 µm; Temperaturprogramm: von 60°C mit 2,5°C/min bis 240°C). Er hatte folgende Zusammensetzung (GC-FI%):
Leichtsieder: 31,1%
Isopropylcyclohexylmethanol: 60,2%
Methoxyisopropylcyclohexylmethanol: 5,5%
Methoxycuminalkohol: 1,6%.

### Beispiel 2: Hydrierung einer Mischung aus Cuminaldehyd (Va) und Methoxycuminaldehyd (Vb)

In einem 300 mL Druckreaktor wurden 4,5 g eines gemäß DE102005029200A1 hergestellten Katalysators in einem Katalysator-Einsatzkorb vorgelegt und mit 100 g Methoxycuminaldehyd (Formel Vb) /Cuminaldehyd (Formel Va) 90%ig im Verhältnis 1:1 versetzt. Die Hydrierung wurde mit reinem Wasserstoff bei einem konstanten Druck von 200 bar und einer Temperatur von 180°C durchgeführt. Es wurde 24 Stunden hydriert. Der Reaktor wurde anschließend entspannt. Der Austrag wurde gaschromatographisch untersucht (GC-Säule: DB-Wax, Länge 30 m, Schichtdicke 0,25 µm; Temperaturprogramm: von 60°C mit 2,5°C/min bis 240°C). Er hatte folgende Zusammensetzung (GC-FI%):
Leichtsieder: 35,8%
Isopropylcyclohexylmethanol: 38,1%
Isopropylbenzylalkohol: 7,8%
Methoxyisopropylcyclohexylmethanol: 1,4%
Methoxycuminalkohol: 1,1%.

## Patentansprüche

1. Verfahren zur Herstellung von 4-Isopropylcyclohexylmethanol der Formel (I) 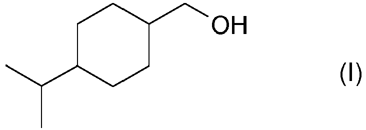 umfassend die Schritte
a) elektrochemische anodische Methoxylierung **dadurch gekennzeichnet, dass** man die elektrochemische anodische Methoxylierung mit mindestens zwei Verbindungen der Formeln (IIa), (IIb), (IIIa) und/oder (IIIb) 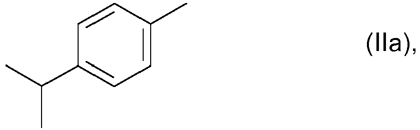 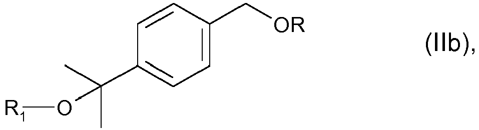 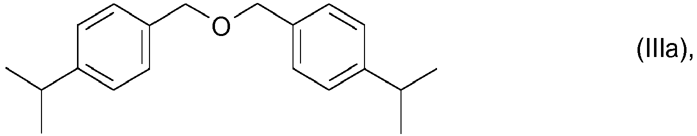 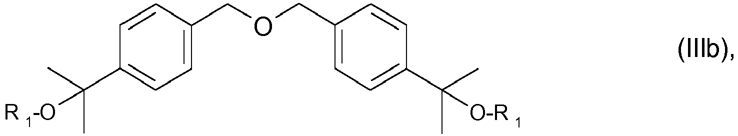 so durchführt, dass eine Mischung der Diacetale der Formeln (IVa) und (IVb) 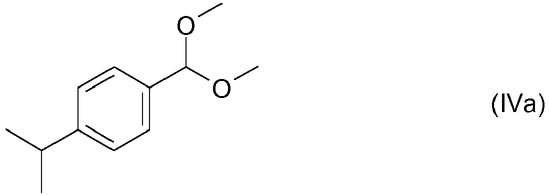 und 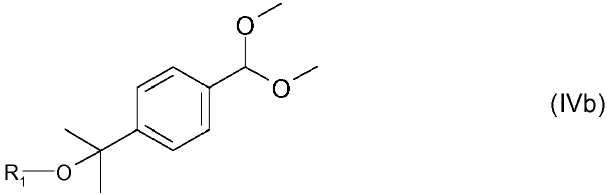 gebildet wird, wobei R₁ in den Formeln (IIb), (IIIb) und (IVb) einen geradkettigen oder verzweigten Alkylrest mit 1 bis 6 Kohlenstoffatomen bedeutet, und R in Formel (IIb) Methyl oder -C(O)R' ist, und R' einen geradkettigen oder verzweigten Alkylrest mit 1 bis 6 Kohlenstoffatomen bedeutet,
b) die Mischung der Diacetale der Formeln (IVa) und/oder (IVb) hydrolysiert unter Bildung der Aldehyde der Formeln (Va) und/oder (Vb) 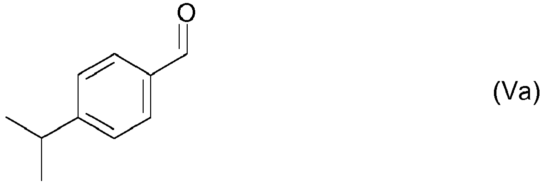 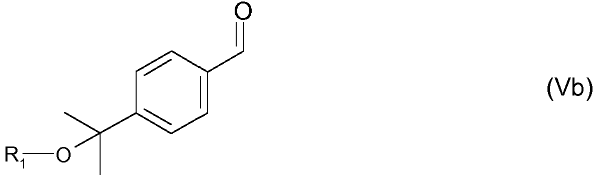 wobei R₁ einen geradkettigen oder verzweigten Alkylrest mit 1 bis 6 Kohlenstoffatomen bedeutet, und
c) das Gemisch der Aldehyde der Formeln (Va) und (Vb) hydriert zu 4-Isopropylcyclohexlmethanol der Formel (I) in Gegenwart von Wasserstoff oder einem wasserstoffhaltigen Gas an einem Katalysator der als Aktivmetall mindestens ein Edelmetall der Gruppe VIII des Periodensystems auf einem Träger enthält.

2. Verfahren gemäß Anspruch 1, **dadurch gekennzeichnet, dass** als Edelmetall der Gruppe VIII des Periodensystems Ruthenium eingesetzt wird.

3. Verfahren gemäß einem der Ansprüche 1 bis 2, **dadurch gekennzeichnet, dass** der Träger Siliciumdioxid enthält.

4. Verfahren gemäß einem der Ansprüche 1 bis 3, **dadurch gekennzeichnet, dass** man die elektrochemische anodische Methoxylierung in Gegenwart der Verbindung der Formel (IIa) 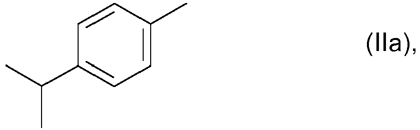 durchführt.

5. Verfahren gemäß einem der Ansprüche 1 bis 4, **dadurch gekennzeichnet, dass** man als Leitsalz Methyltributylammoniummethylsulfat, Methyltriethylammoniummethylsulfat und/oder Schwefelsäure, vorzugsweise Methyltributylammoniummethylsulfat, einsetzt und die Konzentration des Leitsalzes in der Elektrolyselösung im Bereich von 0,1 bis 20 Gewichtsprozent (Gew.-%) wählt.

6. Verfahren gemäß einem der Ansprüche 1 bis 5, **dadurch gekennzeichnet, dass** man als weiteres Lösungsmittel Dimethylcarbonat oder Propylencarbonat oder ein Gemisch dieser Lösungsmittel einsetzt.

7. Verfahren gemäß einem der Ansprüche 1 bis 6, **dadurch gekennzeichnet, dass** man die elektrochemische anodische Methoxylierung bei einer Temperatur der Elektrolyselösung im Bereich von 35 bis 70°C, bei einem Absolutdruck im Bereich von 500 bis 100000 mbar und bei einer Stromdichte im Bereich von 10 bis 100 mA/cm² durchführt.

8. Verfahren gemäß einem der Ansprüche 1 bis 7, **dadurch gekennzeichnet, dass** man die elektrochemische anodische Methoxylierung kontinuierlich und unter Verwendung einer Plattenstapelzelle durchführt.

9. Verfahren gemäß Anspruch 1 **dadurch gekennzeichnet, dass** außer den Aldehyden der Formeln (Va) und (Vb), wobei R₁ Methyl bedeutet auch die entsprechenden Alkohole 4-Isopropylbenzylalkohol und 4-(1-Methoxy-1-methyl-ethyl)-benzylalkohol eingesetzt werden können.

10. Verfahren gemäß Anspruch 1 **dadurch gekennzeichnet, dass** das Massenverhältnis der beiden Aldehyde der Formeln (Va) und (Vb) zwischen 0,2 und 10 liegt.

11. Verfahren gemäß Anspruch 1 **dadurch gekennzeichnet, dass** die Hydrierung bei einer Temperatur von 80-200 °C, vorzugsweise zwischen 120 und 160°C, durchgeführt wird.

12. Verfahren gemäß einem der Ansprüche 1 bis 11 **dadurch gekennzeichnet, dass** die Hydrierung mit Wasserstoff bei einem Gesamtdruck von 100-200 bar durchgeführt wird.

13. Verfahren gemäß einem der Ansprüche 1 bis 12 **dadurch gekennzeichnet, dass** man einen Festbettkatalysator einsetzt, der bezogen auf das Gesamtgewicht des fertigen Katalysators einen Aktivgehalt, insbesondere eine Rutheniumgehalt, von 0,1 bis 0,5 Gew.-% aufweist.

14. Verfahren gemäß einem der Ansprüche 1 bis 13 **dadurch gekennzeichnet, dass** man bei der Hydrierung als Aktivmetall mindestens ein Edelmetall der Gruppe VIII des Periodensystems, vorzugsweise Rhodium und Ruthenium, entweder alleine oder zusammen mit mindestens einem weiteren Metall der Nebengruppen IB, VIIB oder VIII des Periodensystems der Elemente, aufgebracht auf einen Träger enthaltend Siliziumdioxid als Trägermaterial, einsetzt, wobei die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen.

15. Verfahren nach Anspruch 1 bis 14, **dadurch gekennzeichnet, dass** das Verhältnis cis-4-Isopropylcyclohexylmethanol (Ia) zu trans-4-Isopropylcyclohexylmethanol (Ib) größer als 1,9 ist

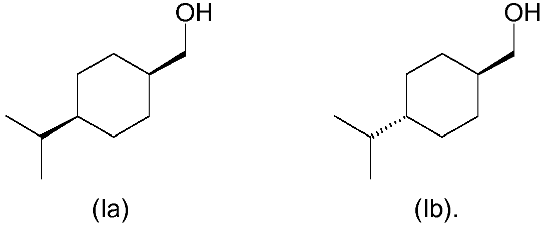

## Claims

1. A process for preparing 4-isopropylcyclohexylmethanol of the formula (I) 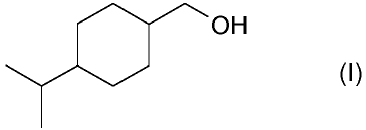 comprising the steps of
a) electrochemical anodic methoxylation, wherein
the electrochemical anodic methoxylation is performed with at least two compounds of the formulae (IIa), (IIb), (IIIa) and/or (IIIb) 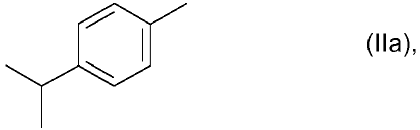 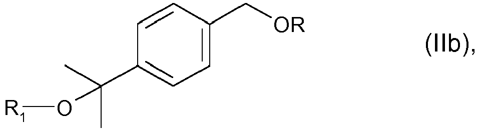 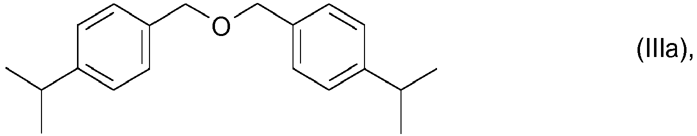 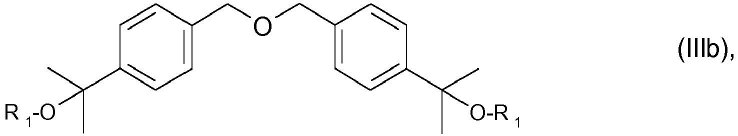 such that a mixture of the diacetals of the formulae (IVa) and (IVb) 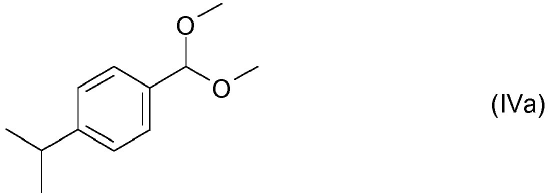 and 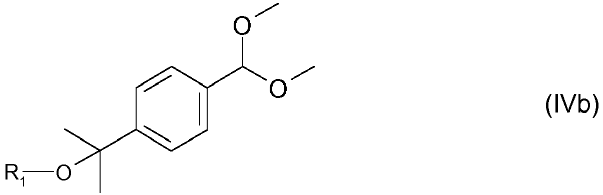 is formed, where R₁ in the formulae (IIb), (IIIb) and (IVb) is a straight-chain or branched alkyl radical having 1 to 6 carbon atoms, and R in formula (IIb) is methyl or -C(O)R', and R' is a straight-chain or branched alkyl radical having 1 to 6 carbon atoms,
b) the mixture of the diacetals of the formulae (IVa) and/or (IVb) is hydrolyzed to form the aldehydes of the formulae (Va) and/or (Vb) 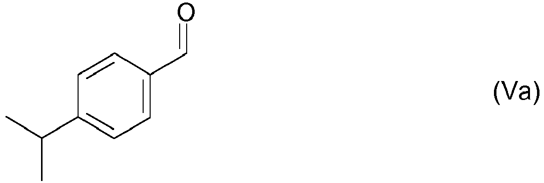 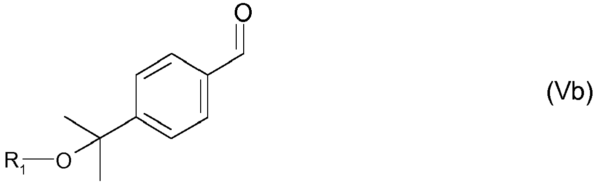 where R₁ is a straight-chain or branched alkyl radical having 1 to 6 carbon atoms, and
c) the mixture of the aldehydes of the formulae (Va) and (Vb) is hydrogenated to 4-isopropylcyclohexylmethanol of the formula (I) in the presence of hydrogen or a hydrogenous gas over a catalyst which comprises, as an active metal, at least one noble metal of group VIII of the periodic table on a support.

2. The process according to claim 1, wherein the noble metal of group VIII of the periodic table used is ruthenium.

3. The process according to either of claims 1 and 2, wherein the support comprises silicon dioxide.

4. The process according to any of claims 1 to 3, wherein the electrochemical anodic methoxylation is performed in the presence of the compound of the formula (IIa)

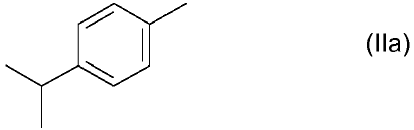

5. The process according to any of claims 1 to 4, wherein the conductive salt used is methyltributylammonium methylsulfate, methyltriethylammonium methylsulfate and/or sulfuric acid, preferably methyltributylammonium methylsulfate, and the concentration of the conductive salt in the electrolysis solution is selected within the range from 0.1 to 20 percent by weight (% by weight).

6. The process according to any of claims 1 to 5, wherein a further solvent used is dimethyl carbonate or propylene carbonate, or a mixture of these solvents.

7. The process according to any of claims 1 to 6, wherein the electrochemical anodic methoxylation is performed at a temperature of the electrolysis solution in the range from 35 to 70°C, at an absolute pressure in the range from 500 to 100 000 mbar and at a current density in the range from 10 to 100 mA/cm².

8. The process according to any of claims 1 to 7, wherein the electrochemical anodic methoxylation is performed continuously and using a plate stack cell.

9. The process according to claim 1, wherein, apart from the aldehydes of the formulae (Va) and (Vb) where R₁ is methyl, it is also possible to use the corresponding alcohols 4-isopropylbenzyl alcohol and 4-(1-methoxy-1-methylethyl)benzyl alcohol.

10. The process according to claim 1, wherein the mass ratio of the two aldehydes of the formulae (Va) and (Vb) is between 0.2 and 10.

11. The process according to claim 1, wherein the hydrogenation is performed at a temperature of 80-200°C, preferably between 120 and 160°C.

12. The process according to any of claims 1 to 11, wherein the hydrogenation with hydrogen is performed at a total pressure of 100-200 bar.

13. The process according to any of claims 1 to 12, wherein a fixed bed catalyst which, based on the total weight of the finished catalyst, has an active content, especially a ruthenium content, of 0.1 to 0.5% by weight is used.

14. The process according to any of claims 1 to 13, wherein the active metal used in the hydrogenation is at least one noble metal of group VIII of the periodic table, preferably rhodium and ruthenium, either alone or together with at least one further metal of transition groups IB, VIIB or VIII of the periodic table of the elements, applied to a support comprising silicon dioxide as a support material, where the amount of the active metal is < 1% by weight, based on the total weight of the catalyst, and at least 60% by weight of the active metal is present in the shell of the catalyst down to a penetration depth of 200 µm.

15. The process according to claims 1 to 14, wherein the ratio of cis-4-isopropylcyclohexylmethanol (Ia) to trans-4-isopropylcyclohexylmethanol (Ib) is greater than 1.9

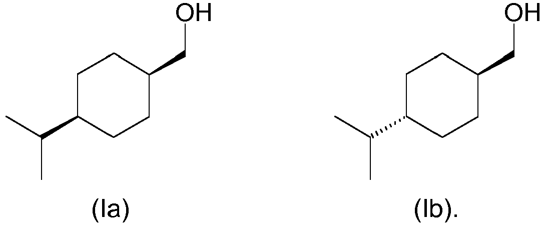

## Revendications

1. Procédé de fabrication de 4-isopropylcyclohexylméthanol de formule (I) 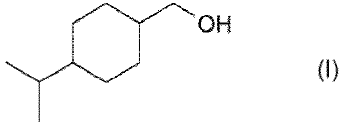 comprenant les étapes suivantes :
a) une méthoxylation anodique électrochimique, **caractérisé en ce que**
la méthoxylation anodique électrochimique est réalisée avec au moins deux composés de formule (IIa), (IIb), (IIIa) et/ou (IIIb) 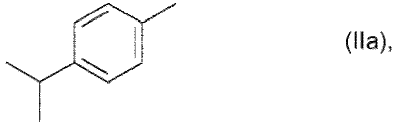 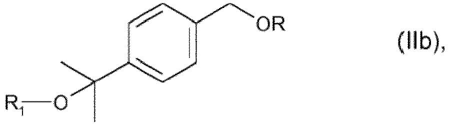 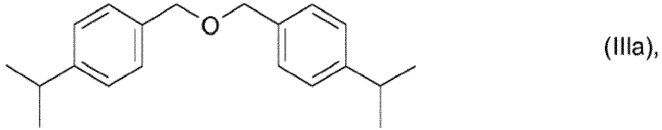 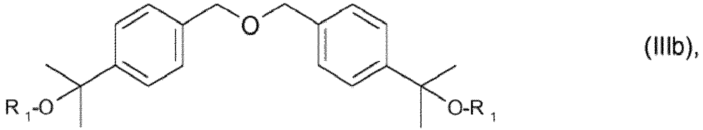 de telle sorte qu'un mélange des diacétals de formule (IVa) et (IVb) 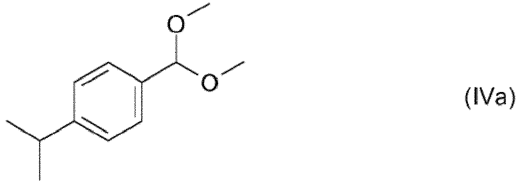 et 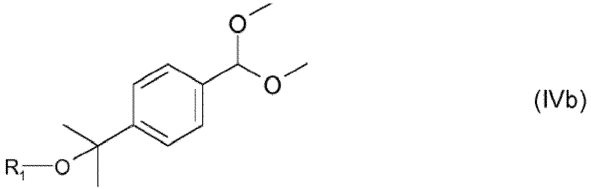 soit formé, R₁ dans les formules (IIb), (IIIb) et (IVb) signifiant un radical alkyle linéaire ou ramifié de 1 à 6 atomes de carbone, et R dans la formule (IIb) étant un méthyle ou -C(O)R', et R' signifiant un radical alkyle linéaire ou ramifié de 1 à 6 atomes de carbone,
b) le mélange des diacétals de formule (IVa) et/ou (IVb) est hydrolysé avec formation des aldéhydes de formule (Va) et/ou (Vb) 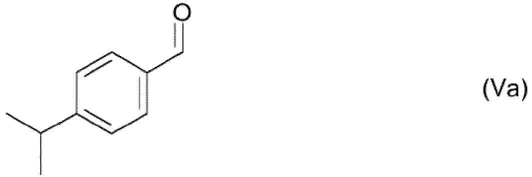 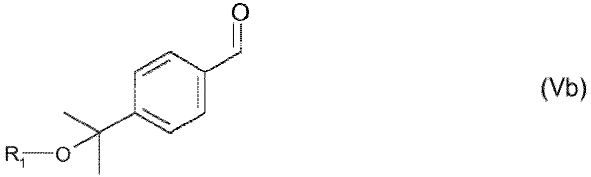 dans lesquelles R₁ signifie un radical alkyle linéaire ou ramifié de 1 à 6 atomes de carbone, et
c) le mélange des aldéhydes de formule (Va) et (Vb) est hydrogéné en 4-isopropylcyclohexylméthanol de formule (I) en présence d'hydrogène ou d'un gaz contenant de l'hydrogène sur un catalyseur qui contient en tant que métal actif au moins un métal noble du groupe VIII du tableau périodique sur un support.

2. Procédé selon la revendication 1, **caractérisé en ce que** le ruthénium est utilisé en tant que métal noble du groupe VIII du tableau périodique.

3. Procédé selon l'une quelconque des revendications 1 à 2, **caractérisé en ce que** le support contient du dioxyde de silicium.

4. Procédé selon l'une quelconque des revendications 1 à 3, **caractérisé en ce que** la méthoxylation anodique électrochimique est réalisée en présence du composé de formule (IIa)

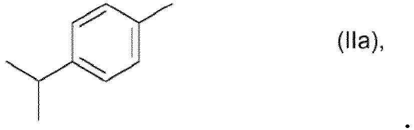

5. Procédé selon l'une quelconque des revendications 1 à 4, **caractérisé en ce que** le méthylsulfate de méthyltributylammonium, le méthylsulfate de méthyl-triéthylammonium et/ou l'acide sulfurique, de préférence le méthylsulfate de méthyltributylammonium, est utilisé en tant que sel conducteur, et la concentration du sel conducteur dans la solution d'électrolyse est choisie dans la plage allant de 0,1 à 20 pour cent en poids (% en poids).

6. Procédé selon l'une quelconque des revendications 1 à 5, **caractérisé en ce que** du carbonate de diméthyle ou du carbonate de propylène ou un mélange de ces solvants est utilisé en tant que solvant supplémentaire.

7. Procédé selon l'une quelconque des revendications 1 à 6, **caractérisé en ce que** la méthoxylation anodique électrochimique est réalisée à une température de la solution d'électrolyse dans la plage allant de 35 à 70 °C, à une pression absolue dans la plage allant de 500 à 100 000 mbar et à une densité de courant dans la plage allant de 10 à 100 mA/cm².

8. Procédé selon l'une quelconque des revendications 1 à 7, **caractérisé en ce que** la méthoxylation anodique électrochimique est réalisée en continu et en utilisant une cellule à empilement de plaques.

9. Procédé selon la revendication 1, **caractérisé en ce qu'**en plus des aldéhydes de formule (Va) et (Vb) dans lesquelles R₁ signifie méthyle, les alcools correspondants alcool 4-isopropylbenzylique et alcool 4-(1-méthoxy-1-méthyl-éthyl)-benzylique peuvent également être utilisés.

10. Procédé selon la revendication 1, **caractérisé en ce que** le rapport massique entre les deux aldéhydes de formule (Va) et (Vb) est compris entre 0,2 et 10.

11. Procédé selon la revendication 1, **caractérisé en ce que** l'hydrogénation est réalisée à une température de 80 à 200 °C, de préférence comprise entre 120 et 160 °C.

12. Procédé selon l'une quelconque des revendications 1 à 11, **caractérisé en ce que** l'hydrogénation est réalisée avec de l'hydrogène à une pression totale de 100 à 200 bar.

13. Procédé selon l'une quelconque des revendications 1 à 12, **caractérisé en ce qu'**un catalyseur en lit fixe est utilisé, qui présente, par rapport au poids total du catalyseur fini, une teneur en agent actif, notamment une teneur en ruthénium, de 0,1 à 0,5 % en poids.

14. Procédé selon l'une quelconque des revendications 1 à 13, **caractérisé en ce qu'**au moins un métal noble du groupe VIII du tableau périodique, de préférence le rhodium et le ruthénium, seul ou conjointement avec au moins un autre métal des groupes de transition IB, VIIB ou VIII du tableau périodique des éléments, appliqué sur un support contenant du dioxyde de silicium en tant que matériau support, est utilisé en tant que métal actif lors de l'hydrogénation, la quantité du métal actif étant < 1 % en poids, par rapport au poids total du catalyseur, et au moins 60 % en poids du métal actif se trouvant dans l'enveloppe du catalyseur jusqu'à une profondeur de pénétration de 200 µm.

15. Procédé selon les revendications 1 à 14, **caractérisé en ce que** le rapport entre le cis-4-isopropylcyclohexylméthanol (Ia) et le trans-4-isopropylcyclohexylméthanol (Ib) est supérieur à 1,9
